Europäisches Patentamt

European Patent Office

Office européen des brevets

(11) Publication number: **0 127 435 B1**

# EUROPEAN PATENT SPECIFICATION

(45) Date of publication of patent specification: **28.08.91**   (51) Int. Cl.⁵: **A61K 31/18**, C07C 303/00

(21) Application number: **84303487.7**

(22) Date of filing: **23.05.84**

(54) Antiarrhythmic class III process.

(30) Priority: **23.05.83 US 497368**

(43) Date of publication of application:
**05.12.84 Bulletin 84/49**

(45) Publication of the grant of the patent:
**28.08.91 Bulletin 91/35**

(84) Designated Contracting States:
**AT BE CH DE FR GB IT LI LU NL SE**

(56) References cited:
**US-A- 3 341 584**

(73) Proprietor: **Bristol-Myers Squibb Company**
**345 Park Avenue**
**New York, N.Y. 10154(US)**

(72) Inventor: **Simon, Arthur**
**52 Tamarack Lane**
**Pomona New York 10970(US)**
Inventor: **Thomis, Jeff A.**
**Opperstraat 266**
**B-1770 Liedekerke(BE)**

(74) Representative: **Jones, Alan John et al**
**CARPMAELS & RANSFORD 43 Bloomsbury**
**Square**
**London, WC1A 2RA(GB)**

## Description

This invention relates to a process for treating arrhythmias, and more particularly, to a process for preventing or ameliorating arrhythmia by lengthening cardiac cell action potential duration and refractory period without beta-adrenergic blockade by administering an effective dose of dextrorotatory 4-(2-isopropylamino-1-hydroxyethyl)methanesulfonanilide or a pharmaceutically acceptable acid addition salt thereof.

The racemic form of 4-(2-isopropylamino-1-hydroxyethyl)methanesulfonanilide, disclosed and claimed in Larsen, et al., U.S. Patent No. 3,341,584, is a recognized beta-blocking agent known in the biological literature as sotalol or MJ 1999. Pharmacologically, beta-blocking compounds reduce sympathetic excitation of the heart and in this respect are considered antiarrhythmics.

Antiarrhythmic drugs are commonly divided into four classes according to their electrophysiological mode of action. Refer to N. Edvardsson, Current Therapeutic Research, 28, No. 1 Supplement, pages 113S-118S, July, 1980, and Keefe, et al., Drugs, 22, 363-400 (1981) for background information of classification first proposed by E. M. Vaughn Williams: classification of antiarrhythmic drugs, in "Symposium of Cardiac Arrhythmias", pages 449-472, Sandoe, et al. (Eds.) A. B. Astra, Soederlalje, Sweden (1970).

Classification of Antiarrhythmic Drugs

I. Local Anesthetic Effect
II. Beta-receptor Blockade
III. Prolongation of Action-potential Duration
IV. Calcium Antagonism

Class I agents usually have little or no effect on action potential duration and exert local anesthetic activity directly at cardiac cell membrane. Class II agents show little or no effect on the action potential and exert their effects through competitive inhibition of beta-adrenergic receptor sites thereby reducing sympathetic excitation of the heart. Class III agents are characterized by their ability to lengthen the action potential duration thereby preventing or ameliorating arrhythmias. Class IV agents are those which have an antiarrhythmic effect due to their actions as calcium antagonists.

According to the above classification, sotalol is a class III antiarrhythmic agent. N. Edvardsson, et al, European Heart Journal, 1, 335-343 (1980); N. Edvardsson, supra.; D. E. Ward, et al, Clin. Cardio. 2, 185-191 (1979); D. P. Myburgh, et al, SA Medical Journal, 295-298 (August, 1979); L. D. Davis, et al, Research in Physiology, 99-114, A. Gaggi Publisher, Bologna (1971); B. N. Singh, et al., Br. J. Pharma., 39, 675-687 (1970). Sotalol is also a Class II antiarrhythmic agent in that it reduces sympathetic excitation of the heart by beta-blockade.

The active ingredient of the instant process "dextrorotatory 4-(2-isopropylamino-1-hydroxyethyl)-methanesulfonanilide" and the corresponding levorotatory stereoisomer have been the subject of biological study and representative publications are listed below. As used in the literature and herein, the racemate form of 4-(2-isopropylamino-1-hydroxyethyl)methanesulfonanilide is at times referred to as sotalol or dl-sotalol, the dextrorotatory isomer as d-sotalol or (+)-sotalol, and the levorotatory isomer as l-sotalol or (-)-sotalol.

D. C. Kvam, et al., J. Pharm. Exper. Therap., 149(2), 183-192 (1965) reported that l-sotalol was about 20-30 times more potent than d-sotalol in preventing certain metabolic effects such as epinephrine-induced hyperglycemia or hyperlipidemia.

J. V. Levy, et al., Proc. Soc. Exp. Biol. Med. 122, 373-379 (1966) studied the inotropic and chronotropic effects of sotalol, d-sotalol, and l-sotalol on rabbit heart atrial preparations and determined that, compared to the racemate, d-sotalol was substantially weaker as a beta-blocking agent whereas l-sotalol was considerably more potent.

P. Somani, et al., J. Pharm. Exper. Therap., 164(2), 317-325 (1968) investigated the antiarrhythmic activity of dextro-and levo- rotatory isomers of sotalol in the dog and found that l-sotalol, considered the active isomer in terms of blockade of beta-receptors, is also the active isomer for specific antiarrhythmic activity (i.e. blockade of adrenergically-induced arrhythmia-Class II). Cardiac arrhythmias induced by ouabain or coronary artery ligation were not suppressed by either isomer demonstrating a lack of non-specific antiarrhythmic activity (Class I) seen with other beta-adrenergic blocking agents such as the levorotatory and dextrorotatory isomers of pronetholol, propranolol and H56/28. The authors concluded that the antiarrhythmic effects of sotalol are a reflection of the specific beta-receptor blocking action of the drug.

Thus, with respect to antiarrhythmic use, there is little in the prior art which would suggest that d-sotalol effectively lengthens cardiac cell action potential duration given the relative inactivity of d-sotalol as a beta-blocking agent.

The present invention is based upon the discovery that d-sotalol lengthens the action potential duration of cardiac cells and is thereby useful in treating heart arrhythmias.

The use as a medicament of d-sotalol essentially free of l-sotalol has not hitherto been disclosed and, therefore, the invention resides in the use of d-sotalol essentially free of l-sotalol for the manufacture of a medicament for prolonging the action potential duration.

Administration of d-sotalol can be carried out orally or parenterally (e.g., intravenous injection) employing liquid or solid form pharmaceutical preparations containing d-sotalol as free base or in the form of a pharmaceutically acceptable acid addition salt in combination with a pharmaceutically acceptable carrier.

The dosage administered depends upon the age, state of health, the weight of the recipient, the extent of the disease, the nature of further treatments possible carried out simultaneously and frequency of the treatment. Usually, the effective dose of d-sotalol ranges from 0.3-8.6 mg/kg body weight of said mammal. In the case of a human, a dose of from 20 to 600 mg per patient is given from 1 to 4 times per day with oral administration preferred. When d-sotalol is administered by the preferred oral route, a larger quantity of d-sotalol is required, preferably 160-480 mg once or twice/day, to produce the same effect as a smaller quantity given parenterally, for example by intravenous injection. The d-sotalol is administered in accordance with good clinical practice, that is, d-sotalol is administered at an effective dose that will produce an increase in action potential duration without causing any harmful or untoward side effects.

Conventional techniques for studying arrhythmias including ambulatory electrocardiography with computer-assisted analysis and programmed stimulation techniques for arrhythmia induction during intracardiac electrophysiological study are employed to determine effectiveness of a specific dose of d-sotalol in treating arrhythmias by prolongation of action potential duration. N. Edvardsson, et al., supra.

Pharmaceutically acceptable acid addition salts of d-sotalol are prepared in conventional manner known in the art, for example, by solution of d-sotalol in a suitable solvent and addition of the desired acid, for example, in a stoichiometric ratio, and isolation of the salt by standard techniques such as concentration and crystallization. Examples of pharmaceutically acceptable acid addition salts of d-sotalol which may be prepared in this manner include salts of inorganic acids such as sulfuric, nitric, phosphoric, and preferably hydrochloric acid, as well as organic acids such as acetic, propionic, succinic, furmaric, maleic, citric, tartaric, cinnamic, lactic, mandelic, ethanedisulfonic acid and the like.

The pharmaceutical compositions of d-sotalol employed in the process of the instant invention can be prepared in the conventional way using the common carriers, bindary auxiliaries, and solvents. As previously stated, oral administration is preferred and dosage forms compatible therewith are employed. Compositions suitable for oral administration include conventionally prepared solutions, tablets, capsules, drages, etc. prepared from standard pharmaceutical excipients and carriers such as mannitol, milk sugar, organic or inorganic calcium salts, etc., binders such as polyvinylpyrrolidone, gelatin, or cellulose derivatives, as were tablet dissolving agents such as starch or alginic acid, lubricants such as stearic acid, and inorganic flow agents such as talc or colloidal salicylic acid.

EXAMPLE 1

Resolution of 4-(2-Isopropylamino-1-hydroxyethyl)methanesulfonanilide

d-Sotalol·l-mandelate.- A solution of racemic sotalol (24.5 g., 0.09 mole) (obtained by neutralizing sotalol hydrochloride in ethanol with a mole equivalent of concentrated sodium hydroxide, concentration and extraction of the free base in acetonitrile) in 200 ml. of hot isopropanol was mixed with 13.7 g. (0.09 mole) of l-mandelic acid. On cooling, an optically enriched fraction, 26.0 g., m.p. 125-140°, $[\alpha]_D^{25}$ -27.2°, of the d-sotalol·l-mandelate salt was obtained. Crystallization from isopropanol (300 ml.) afforded 18.7 g., m.p. 139-145.5°, $[\alpha]_D^{25}$ -25.4°. Further recrystallization of this material from 1:1 isopropanol-absolute ethanol provided d-sotalol·l-mandelate as white fluffy needles, m.p. 154.5-156°, $[\alpha]_D^{25}$ -14.2°.

Anal. Calcd. for $C_{12}H_{20}N_2O_3S \cdot C_8H_8O_3$: C, 56.58; H, 6.65; N, 6.60. Found: C, 56.71; H, 6.82; N, 6.51.

d-Sotalol Hydrochloride.- Acidification of a suspension of d-sotalol·l-mandelate (10.6 g., 0.025 mole $[\alpha]_D^{25}$ -14.2°) in 150 ml. of isopropanol with 8 ml. of 3.9N ethanolic hydrogen chloride afforded complete solution at reflux temperature. On cooling 7.0 g. (90%) of a white crystalline solid deposited which after crystallization from 20 ml. of methanol and 150 ml. of isopropanol provided 6.0 g. (78%) of analytical product, m.p. 204-205.5° (dec.), $[\alpha]_D^{25}$ +36.0°.

Anal. Calcd. for $C_{12}H_{20}N_2O_3S \cdot HCl$: C, 46.67; H, 6.85; Cl, 11.48. Found: C, 46.81; H, 6.98; Cl, 11.44.

EXAMPLE 2

3

Electrophysiological Effects of Sotalol, d-Sotalol and l-Sotalol

Perfused cardiac Purkinje fibers and guinea pig cardiac papillary muscle were stimulated electrically and transmembrane potentials recorded with glass microelectrodes in conventional manner known to the art. L. D. Davis, et al., Research In Physiology, Ed. F. F. Kao, et al., page 99, A. Gaggi, Bologna, 1971.

Evaluation of the test agent was carried out by increasing the concentration in successive steps from 3 x $10^{-7}$M up to 3 x $10^3$M with each concentration applied during a 30 min. period. The preparations were stimulated at 60/min. and transmembrane potentials measured using standard micro-electrode technique.

At concentration between 3 X $10^{-7}$M and $10^{-4}$M, sotalol, d-sotalol and l-sotalol significantly prolonged the action potential duration with nearly identical effects.

EXAMPLE 3

d-Sotalol Class III Action in the Dog

The effects of d-sotalol at $10^{-6}$ to 5 x $10^{-4}$M on the action potentials of ventricular muscle and Purkinge fibers from infarcted (Inf.) and non-infarcted (Non-Inf.) areas was determined in 10 dogs four days after coronary ligation with the following effects at 5 x $10^{-4}$M concentration shown as mean ± standard deviation.

| | Ventricular | | Purkinge | |
|---|---|---|---|---|
| | Non-Inf. | Inf. | Non-Inf. | Inf. |
| **Action Potential Duration:** | | | | |
| Control | 219 ± 41 | 173 ± 49 | 278 ± 42 | 338 ± 42 |
| d-sotalol | 250 ± 44* | 201 ± 53* | 372 ± 39* | 419 ± 68* |
| **Effective Refractory Period:** | | | | |
| Control | 215 ± 24 | 230 ± 37 | 220 ± 37 | 248 ± 44 |
| d-Sotalol | 244 ± 53* | 294 ± 36* | 302 ± 44* | 367 ± 52* |

\* p below 0.05 d-sotalol vs. control.

The data demonstrates that in both non-infarcted and infarcted areas, d-sotalol significantly prolonged the action potential duration and that the effective refractory period was significantly more prolonged in the infarcted compared to the non-infarcted areas leading to the conclusion that d-sotalol has significant Class III effects.

**Claims**

1. The use of d-sotalol essentially free of l-sotalol for the manufacture of a medicament for prolonging the action-potential duration.

2. The use of claim 1 wherein the medicament is in oral dosage form.

3. The use of Claim 1 or claim 2 wherein the medicament is in unit dosage form, comprises from 160 to

4

600 mg of d-sotalol and is formulated for administration to a human patient.

4. The use of Claim 3 wherein the medicament comprises from 160 to 480 mg of d-sotalol.

5. The use of any one of Claims 1 to 4 wherein the d-sotalol is in the form of a pharmaceutically acceptable acid addition salt thereof.

6. The use of Claim 5 wherein the d-sotalol is in the form of d-sotalol hydrochloride.

7. A process of preparing a medicament for prolonging the action potential duration, comprising formulating d-sotalol essentially free of l-sotalol and optionally a pharmaceutically acceptable carrier for use in prolonging the action potential duration.

8. The process of Claim 7 wherein the medicament is as further defined in any one of Claims 2 to 6.

## Revendications

1. Utilisation, pour la préparation d'un médicament destiné à prolonger la durée du potentiel d'action du d-sotalol, pratiquement exempt de l-sotalol, .

2. Utilisation selon la revendication 1, dans laquelle le médicament se présente sous une forme posologique orale.

3. Utilisation selon les revendications 1 ou 2, dans laquelle le médicament se présente sous une forme posologique unitaire, comprend de 160 à 600 mg de d-sotalol et est formulé pour être administré à un patient humain.

4. Utilisation selon la revendication 3, dans laquelle le médicament comprend de 160 à 480 mg de d-sotalol.

5. Utilisation selon l'une quelconque des revendications 1 à 4, dans laquelle le d-sotalol se présente sous la forme d'un de ses sels d'addition d'acide, pharmaceutiquement acceptable.

6. Utilisation selon la revendication 5, dans laquelle le d-sotalol se présente sous la forme de chlorhydrate de d-sotalol.

7. Procédé de préparation d'un médicament destiné à prolonger la durée du potentiel d'action, qui consiste à formuler du d-sotalol pratiquement exempt de l-sotalol et, éventuellement, un excipient pharmaceutiquement acceptable, pour prolonger la durée du potentiel d'action.

8. Le procédé selon la revendication 7, dans lequel le médicament est tel que défini plus en détail dans l'une quelconque des revendications 2 à 6.

## Patentansprüche

1. Die Verwendung von im wesentlichen l-sotalolfreiem d-Sotalol für die Herstellung eines Medikaments zur Verlängerung der Aktionspotentialdauer.

2. Verwendung nach Anspruch 1, wobei das Medikament in einer oralen Dosierungsform vorliegt.

3. Verwendung nach Anspruch 1 oder 2, wobei das Medikament in Dosierungseinheiten vorliegt, die 160 bis 600 mg d-Sotalol enthalten, und für die Verabreichung an einen menschlichen Patienten formuliert wurde.

4. Verwendung nach Anspruch 3, wobei das Medikament 160 bis 480 mg d-Sotalol enthält.

5. Verwendung nach irgendeinem der Ansprüche 1 bis 4, wobei das d-Sotalol in Form eines seiner pharmzeutisch verträglichen Säureadditionssalze vorliegt.

6. Verwendung nach Anspruch 5, wobei das d-Sotalol als d-Sotalol-Hydrochlorid vorliegt.

7. Verfahren zur Herstellung eines Medikaments zur Verlängerung der Dauer des Aktionspotentials, das die Formulierung von im wesentlichen l-sotalolfreiem d-Sotalol und gegebenenfalls von einem arzneilich resorbierbaren Trägerstoff (Carrier) zum Zwecke der Verwendung bei der Verlängerung der Aktionspotentialdauer umfaßt.

8. Verfahren nach Anspruch 7, bei dem es sich um ein Medikament handelt, wie es in irgendeinem der Ansprüche 2 bis 6 näher definiert ist.